# EUROPÄISCHE PATENTSCHRIFT

(11) **EP 2 470 088 B1**
(45) Veröffentlichungstag und Bekanntmachung des Hinweises auf die Patenterteilung: **14.06.2017**
(21) Anmeldenummer: 10754588.1
(22) Anmeldetag: 25.08.2010
(51) Int. Cl.: A61B 17/3207, A61B 17/221, A61B 90/00

(54) **KATHETER MIT PROTEKTIONSSYSTEM ZUM ANSAUGEN, FRAGMENTIEREN UND HINAUSFÖRDERN VON ENTFERNBAREM MATERIAL AUS HOHLKÖRPERN BZW. GEFÄSSEN, INSBESONDERE DES MENSCHLICHEN ODER TIERISCHEN KÖRPERS**
CATHETER COMPRISING A PROTECTION SYSTEM FOR ASPIRATING, FRAGMENTING AND EXTRACTING REMOVABLE MATERIAL FROM HOLLOW BODIES AND/OR VESSELS, IN PARTICULAR OF THE HUMAN OR ANIMAL BODY
CATHÉTER AVEC SYSTÈME DE PROTECTION POUR L'ASPIRATION, LA FRAGMENTATION ET L'EXTRACTION DE MATÉRIAU POUVANT ÊTRE ÉLIMINÉ, PRÉSENT DANS DES CAVITÉS OU DES VAISSEAUX, EN PARTICULIER DU CORPS HUMAIN OU DU CORPS ANIMAL

(30) Priorität: 27.08.2009 CH 13282009
(43) Veröffentlichungstag der Anmeldung: 04.07.2012
(73) Patentinhaber: Straub Medical AG, 7323 Wangs (CH)
(72) Erfinder: STRAUB, Immanuel, CH-7323 Wangs (CH); HELLER, Mathias, CH-8352 Elsau (CH)
(74) Vertreter: Patentbüro Paul Rosenich AG
(86) Internationale Anmeldenummer: PCT/IB2010/053816
(87) Internationale Veröffentlichungsnummer: WO 2011/024124

(56) Entgegenhaltungen:
- EP-A1- 1 690 504
- WO-A-00/47116
- WO-A-2005/084562
- US-A- 5 695 519
- US-A- 5 876 414
- US-A1- 2002 123 761
- US-A1- 2003 055 404
- US-A1- 2008 004 646
- US-B1- 6 454 775
- US-B1- 6 579 298

## Beschreibung

Die Erfindung betrifft eine Vorrichtung zum Entfernen und Hinausfördern von entfernbarem Material, wie Ablagerungen aus dem Innern von Hohlkörpern, insbesondere von Thromben und Embolien aus Blutgefässen, mit einem in den Hohlkörper einführbaren Abbaukatheter und einem ebenfalls in den Hohlkörper einführbaren Schleusenkatheter, wobei der Abbaukatheter und der Schleusenkatheter je ein proximales Ende und ein distales Ende aufweisen, der Schleusenkatheter an seinem distalen Endbereich mit einem radial verformbaren, trichterförmigen, sich gegen sein freies, distales Ende im Querschnitt erweiternden Endstück aus flüssigkeitsdurchlässigem Material verbunden ist und der Abbaukatheter einen Arbeitskopf und eine damit verbundene flexible Hülle aufweist, wobei der Arbeitskopf des Abbaukatheters zum Einführen in den Bereich des trichterförmigen Endstückes des Schleusenkatheters einen Aussendurchmesser aufweist, der kleiner als der Innendurchmesser des erweiterten Endstückes ist. Weiter betrifft die Erfindung einen Abbaukatheter für eine derartige Vorrichtung.

Ablagerungen, z. B. in der Form von Thromben und Embolien in Blutgefässen, verringern den Durchflussquerschnitt von Hohlkörpern, wie Adern, Arterien und Venen. Dies kann zu Stauungen oder zu einem totalen Verschluss (Embolien) des Blutgefässes führen. Die gesundheitlichen Folgen davon können Durchblutungsstörungen oder im Extremfall der Ausfall von Gliedmassen bzw. sogar von lebenswichtigen Organen sein.

Unter Hohlkörpern sind insbesondere auch zu verstehen: Bypässe, Stents oder dgl., aber auch Hohlorgane, wie Blase, Niere, Lunge, Herz und Uterus.

Aus der WO-A-2005/084562 ist ein Katheter, insbesondere zum Entfernen von frischen Blutgerinnseln bekannt, der einen Arbeitskopf mit einem Stator und einem darin rotierenden, mit einer Förderschraube verbundenen Rotor aufweist. Die Handelsmarke dieses Katheters ist Aspirex ® (Marke der Anmelderin). Der Stator ist an seinem Umfang mit wenigstens einer Eintrittsöffnung versehen. Durch den von der Förderschraube erzeugten Unterdruck werden die abzutragenden Ablagerungen durch die Eintrittsöffnungen in den Stator angesaugt, wo sie durch die scherende Wirkung zwischen dem Stator und der Förderschraube fragmentiert und schliesslich über die Förderschraube abgeführt werden. Vor allem bei inhomogenen Ablagerungen kann es vorkommen, dass einzelne Partikel mit dem Blutfluss abgeschwemmt werden und es bei weiter entfernteren Stellen, wo die Gefässlumen kleiner sind, zu erneuten Stauungen kommt.

Die US-A-5,102,415 zeigt eine Vorrichtung zum mechanischen Entfernen von Blutgerinnseln aus Venen und Arterien. Dabei wird zunächst ein Aussenkatheter über einen Führungsdraht bis vor den Thrombus in das Blutgefäss eingeführt. Anschliessend wird ein Schleusenkatheter durch den Aussenkatheter eingeführt. Der Schleusenkatheter ist an seinem distalen Ende mit einem radial aufweitbaren Endstück versehen. Beim Einführen des Schleusenkatheters in den Aussenkatheter wird das Endstück auf den Innendurchmesser des Aussenkatheters reduziert. Nachdem das Endstück durch den Aussenkatheter hindurchgeschoben ist, entfaltet sich das Endstück trichterförmig. Nun wird ein Innenkatheter durch den Schleusenkatheter und durch den Thrombus hindurch eingeführt. Am proximalen Ende des Innenkatheters befindet sich ein Miniaturballon. Nachdem der Innenkatheter den zu entfernenden Thrombus durchdrungen hat, wird der Miniaturballon aufgeblasen. Durch Zurückziehen des Innenkatheters wird nun der Thrombus mechanisch in das trichterförmige Endstück hineingezogen. Beim gemeinsamen Herausziehen des Aussenkatheters mit dem Schleusenkatheter und dem Innenkatheter wird nun der Thrombus aus dem Blutgefäss entfernt. Dieses Verfahren weist den Nachteil auf, dass gelegentlich Reste des Thrombus an der Gefässwand hängen bleiben, die wieder Strömungswiderstände bilden und/oder später abgeschwemmt werden können. Abgeschwemmte Reste können in distalen, engen Blutgefässen zu neuen Verstopfungen führen.

Aus der US-B-6,454,775 ist ein Gerät zum Entfernen von Blutgerinnseln bekannt, das einen flexiblen Katheter und eine motorische Antriebseinheit umfasst. Am distalen Ende des Katheters befindet sich ein radial aufweitbarer Arbeitskopf mit federnden Drähten. Durch die Rotation des Arbeitskopfes sollen die Ablagerungen abgelöst und entfernt werden.

In Fig. 9 zeigt die US-B-6,454,775 einen zweiten Katheter, der von der Gegenseite her in das verstopfte Blutgefäss eingeschoben wird. Dieser zweite Katheter ist an seinem distalen Ende mit einem radial aufweitbaren trichterförmigen Endstück versehen. Die mittels des Arbeitskopfes abgelösten und mit dem Blutfluss abgeschwemmten Ablagerungen sollen offenbar im trichterförmigen Endstück aufgefangen und dann von der Antriebseinheit abgesaugt werden.

Das Entfernen von Thromben mit diesem System ist relativ aufwändig, da die entfernten Ablagerungen aus grösseren und kleineren Bröckchen bestehen, welche tendenziell zusammenkleben, aber nicht zerkleinert werden, und damit die im Blut verbleibenden Teilchen im Blut schweben können. Dies führt zur Klumpenbildung und Verstopfung des Gangsystems.

Aus der US-B-6,579,298 ist eine Vorrichtung zum Entfernen und Hinausfördern von Ablagerungen aus dem Innern von Hohlkörpern mit einem in den Hohlkörper einführbaren Abbaukatheter und einem ebenfalls in denselben Hohlkörper einführbaren Schleusenkatheter, gemäß dem Oberbegriff des Anspruchs 1, bekannt. Der Abbaukatheter und der Schleusenkatheter weisen je ein proximales Ende und ein distales Ende auf. Der Schleusenkatheter ist an seinem distalen Ende mit einem radial verformbaren, trichterförmigen, sich gegen sein distales Ende im Querschnitt erweiternden Endstück aus flüssigkeitsdurchlässigem Material verbunden. Der Abbaukatheter weist einen Arbeitskopf und eine damit verbundene flexible Hülle auf, wobei der Arbeitskopf des Abbaukatheters zum Einführen in den Bereich des trichterförmigen Endstückes des Schleusenkatheters einen Aussendurchmesser aufweist, der kleiner als der Innendurchmesser des erweiterten Endstückes ist. Der Arbeitskopf des Abbaukatheters ist als Fräser ausgebildet.

Zum Entfernen und Hinausfördern von Ablagerungen aus dem Innern von Hohlkörpern wird bei der Vorrichtung gemäss der US-B-6,579,298 der Abbaukatheter und Schleusenkatheter gleichzeitig in den Hohlkörper bis vor das zu entfernende Material eingeführt. Das trichterförmige Endstück des Schleusenkatheters wird dann aufgeweitet, wodurch der Hohlkörper wenigstens teilweise verschlossen wird. Anschliessend wird mit dem Fräser die Ablagerung im Hohlkörper abgebaut, wobei das vom Fräser abgebaute Material durch die flexible Hülle abgesaugt wird.

Der Fräser zerkleinert lediglich die Ablagerungen in Stücke undefinierter Grösse. Somit können Teilstücke des zu entfernenden Materials anfallen, welche die flexible Hülle verstopfen und somit das Entfernen der Ablagerungen behindern können.

Aus der US-A-5876414 ist ein Abbaukatheter gemäß dem Oberbegriff des Anspruchs 10 bekannt. Der Erfindung liegt die Aufgabe zugrunde, eine Vorrichtung zu schaffen, die insbesondere ein rasches und zuverlässiges Entfernen von Thromben aus Blutgefässen ermöglicht, die aber auch aus beliebig anderen Hohlkörpern sicher festes Material abtragen und entfernen kann, ohne Festkörperteile über den unmittelbaren Arbeitsbereich hinaus zu verteilen.

Die Erfindung wird in Anspruch 1 und in Anspruch 10 beschrieben. Gemäss der Erfindung wird dies bei der Vorrichtung, die einen Abbaukatheter und einen Schleusenkatheter aufweist, dadurch erreicht, dass der Arbeitskopf des Abbaukatheters einen hülsenförmigen Stator und einen zentral darin angeordneten, relativ zum Stator in Drehung versetzbaren Rotor aufweist, der Stator an seinem Umfang mit wenigstens einer seitlichen Eintrittsöffnung versehen ist und der Rotor mit einer Antriebs- und Förderschraube verbunden oder einstückig ausgebildet ist, wobei die wenigstens eine seitliche Eintrittsöffnung des Stators als jeweils wenigstens zwei, in Bezug auf die Längsachse des Abbaukatheters axial hintereinander angeordnete kreisrunde Löcher ausgebildet ist.

Das Herstellen der im Wesentlichen kreisrunden Löcher ist mit den üblichen Werkzeugen wie Bohrer, Fräser etc. besonders einfach. Kreisrunde Löcher können aber auch durch Stanzen, Erodieren oder Wasserstrahlschneiden rationell hergestellt werden. Die Kanten, die die Löcher begrenzen, bilden Scherkanten, an denen angesaugtes Material zerkleinert wird. Pro Loch werden, in Längsrichtung des Arbeitskopfes gesehen, zwei bogenförmig verlaufende Scherkanten gebildet. Entsprechend sind bei zwei Löchern vier Scherkanten, bei drei Löchern sechs Scherkanten, usw. vorhanden. Durch diese Ausbildung der wenigstens einen seitlichen Eintrittsöffnung kann der Arbeitskopf des Abbaukatheters sich während des Abbauvorganges nicht im Schleusenkatheter verhaken. Gleichzeitig gewährleisten die Scherkanten der Eintrittsöffnung eine Schneidfunktion des Abbaukatheters, so dass die vom Abbaukatheter abgebauten Ablagerungen derart fragmentiert bzw. zerkleinert werden, dass eine hohe Abbauleistung ohne die Gefahr einer Verstopfung der flexiblen Hülle beim Absaugen des fragmentierten Materials der Ablagerungen durch die flexible Hülle gegeben ist.

Durch die erfindungsgemässe Kombination mit dem Schleusenkatheter kann praktisch ausgeschlossen werden, dass einzelne Partikel des Thrombus durch die Blutzirkulation oder den Blutfluss an weiter entfernte und unter Umständen nicht mehr zugängliche Stellen abgeschwemmt werden. Solche sich im trichterförmigen Endstück des Schleusenkatheters verfangenden Partikel können nun auch mittels des Abbaukatheters selbst aus dem Endstück entfernt werden.

Während also das Endstück des Schleusenkatheters bisher die Funktion des Auffangens und Verwahrens hatte, wird es nunmehr durch die neuartige Kombination mit dem Abbaukatheter nur mehr als Abschwemmbarriere verwendet. Das Endstück des Schleusenkatheters dient sozusagen nur mehr der Sicherheitserhöhung, aber nicht mehr dem eigentlichen Abtransport des abzutransportierenden Materials. Daraus ergibt sich somit eine völlige neue Anwendung des an sich bekannten Endstückes und eine insgesamt wesentlich verbesserte und sicherere Behandlung der Gefässverschlüsse. Das Zusammenwirken der beiden Katheter erzielt somit optimale Ergebnisse bei der Entfernung von Material aus Hohlräumen.

In einer Ausführung der Vorrichtung weist der Schleusenkatheter einen zentralen Kanal auf, durch den der Abbaukatheter so weit einführbar ist, dass der Arbeitskopf des Abbaukatheters im Abbauzustand bis in den Bereich des trichterförmigen Endstückes ragt. Der Schleusenkatheter und der Abbaukatheter werden bei dieser Vorrichtung somit von derselben Seite her, vorzugsweise entgegen dem Blutfluss, in den Hohlkörper eingeführt. Der Abbaukatheter entfernt dabei durch Vorschieben des Abbaukatheters ebenfalls zunächst den Thrombus. Beim anschliessenden Zurückziehen kann dann mit dem Abbaukatheter das trichterförmige Endstück ausgeräumt und die sich darin verfangenen Partikel entfernt werden. Der Vorteil dieses Aufbaus liegt darin, dass der Patient nur an einer Stelle geöffnet werden muss.

Natürlich kann bei diesem Aufbau bzw. bei dieser Anwendung der Erfindung mit dem Abbaukatheter auch bis relativ weit in schmale Gefässe vorgearbeitet werden. Lediglich der Gefässinnendurchmesser wirkt hinsichtlich des Abbaukatheter-Durchmessers begrenzend, der jedoch relativ klein ausgebildet werden kann.

Eine besonders zweckmässige Ausführung besteht darin, dass sich die Kreisumfänge der Löcher überschneiden. Beispielsweise bei zwei Löchern, deren Kreisumfänge sich überschneiden, ergibt sich eine etwa 8-förmige Eintrittsöffnung. Diese Form ist günstig im Zusammenwirken mit dem trichterförmigen Endstück, da ein Verhaken zwischen dem Endstück des Schleusenkatheters und dem Arbeitskopf des Abbaukatheters dadurch zusätzlich weitgehend vermieden wird.

Die Löcher sind vorteilhaft durch einen in axialer Richtung verlaufenden, im Wesentlichen zentrisch zu den Löchern angeordneten Schlitz miteinander verbunden. Ein solcher Schlitz verhindert das Entstehen spitzer Zähne, welche bei Löchern mit sich teilweise überschneidenden Kreisumfängen entstehen können. Zudem werden durch den Schlitz weitere Scherkanten ausgebildet, welche bei der Fragmentierung bzw. Zerkleinerung des vom Abbaukatheter angesaugten Materials vorteilhaft mitwirken.

Zweckmässigerweise erstreckt sich der Schlitz in Richtung zum proximalen Ende des Abbaukatheters axial über die Löcher hinaus. Somit ergibt sich in Verlängerung der Löcher ein gegenüber dem Durchmesser der Löcher schmalerer Kanal. Besonders zähe Teile eines Thrombus, welche im Bereich der Löcher nicht abgeschert werden können, werden in diesen Kanal hineingezogen und spätestens am proximalen Ende des Kanals abgeschert. Die von dieser Schlitzausbildung zusätzlich geschaffenen Scherkanten unterstützen dabei vorteilhaft die Fragmentierung des vom Abbaukatheter angesaugten Materials. Es sei hier nochmals hervorgehoben, dass durch eine ausreichende Zerkleinerung bzw. Fragmentierung des vom Arbeitskopf abgebauten und angesaugten Materials der Abtransport durch die flexible Hülle des Abbaukatheters in vorteilhafter Weise sichergestellt wird und dadurch Komplikationen, welche sich bei einem Operationsvorgang durch einen verstopften Absaugkanal ergeben können, weitgehend ausgeschlossen werden.

Vorteilhaft sind am Stator des Abbaukatheters jeweils zwei einander etwa diametral gegenüberliegend angeordnete, seitliche Eintrittsöffnungen vorgesehen. Zwei einander diametral gegenüberliegend angeordnete Eintrittsöffnungen ergeben eine gleichmässige Kräfteverteilung beim Abbauvorgang und bei entsprechender Leistung der Förderschraube zudem eine höhere Abbauleistung. Des Weiteren stehen somit eine Vielzahl von Scherkanten am Stator zur Verfügung, welche eine ausreichende Fragmentierung des vom Arbeitskopf angesaugten Materials sicherstellen. Besonders vorteilhaft sind die einander gegenüberliegenden, seitlichen Eintrittsöffnungen spiegelbildlich zueinander angeordnet, womit eine noch gleichmässigere Kräfteverteilung beim Abbauvorgang und somit noch verbesserte Abbauleistung gewährleistet ist.

Die Grösse der aus einem Blutgefäss zu entfernenden Thromben kann sehr stark variieren. Es hat sich daher als vorteilhaft erwiesen, dass der Stator des Arbeitskopfes im Aussendurchmesser so abgestuft ist, dass sich dieser zu seinem freien distalen Ende hin verjüngt. Mit dem verjüngten Abschnitt am distalen Ende des Stators wird im Thrombus zuerst eine Art "Kernbohrung" erzeugt, die dann vom nachfolgenden, im Durchmesser vergrösserten Abschnitt "aufgebohrt" wird. Diese neue Form des Stators ermöglicht eine sehr hohe Abbauleistung und führt somit zu kurzen Behandlungszeiten für den Patienten.

Besonders zweckmässig weist das Endstück im aufgeweiteten Zustand des Endstückes eine Sieb- oder Gitterstruktur auf. Diese kann beispielsweise als ein Gewebe aus einem textilen oder metallischen Material bestehen. Wie bereits ausgeführt wurde, hat das trichterförmige Endstück des Schleusenkatheters die Aufgabe, abgeschwemmte Partikel des Thrombus aus dem Blutfluss aufzufangen. Aus diesem Grund ist ein trichterförmiges Endstück aus flüssigkeitsdurchlässigem Material bevorzugt, wodurch der Blutfluss im aufgeweiteten Zustand des Endstückes nur eingeschränkt, aber nicht vollständig gestoppt wird.

Zum Einführen des Schleusenkatheters in den Hohlkörper ist das Endstück vorteilhaft auf den Aussendurchmesser des Schleusenkatheters verjüngt. Hat das Endstück nach dem Einführen in den Hohlkörper seine Position erreicht, so wird das Endstück wieder, vorteilhaft konisch, aufgeweitet. Das Endstück ist daher vorteilhaft schirmartig aufweitbar.

Bei genügender Elastizität bzw. Vorspannung des für das Endstück verwendeten Materials kann sich das Endstück selbstständig auseinanderfalten, sobald das Endstück an der gewünschten Position positioniert ist. Das Endstück ist vorteilhaft mittels eines aufblasbaren Ballons, mittels eines selbstexpandierenden Mechanismus oder mittels des Abbaukatheters radial aufweitbar. Diese unterstützenden Massnahmen ermöglichen auch bei einem grossen Widerstand gegen das Auseinanderfalten des Endstückes, das Endstück auseinanderzufalten bzw. aufzuweiten. Für den Aufbau des Endstückes des Schleusenkatheters kann jede Technik benutzt werden, die dazu führt, dass das positionierte Endstück sich so öffnet, dass das Gefäss bzw. der Hohlkörper (z. B. Stent) partikeldicht (nicht flüssigkeitsdicht) abgeschlossen wird. Insbesondere selbstexpandierende Gitternetzwerke können dafür eingesetzt werden.

Bevorzugt sind die Kanten der Löcher als Schneidkanten ausgebildet, womit eine noch verbesserte Fragmentierung des vom Abbaukatheter angesaugten Materials sichergestellt wird. Die Schneidkanten werden vorteilhaft durch scharfe Kanten gebildet, die der Förderschraube zugewandt am Stator des Arbeitskopfes angeordnet sind. Die Schneidkanten können auch einen strukturierten Verlauf, beispielsweise einen wellenförmigen und/oder gezackten Verlauf aufweisen.

Diese neue Form des Stators des Abbaukatheters kann auch unabhängig vom Schleusenkatheter vorteilhaft eingesetzt werden, weshalb darinnen auch eine eigene Erfindung gesehen wird.

Ein vorteilhaftes nicht beanspruchtes Verfahren zur Entfernung von Material aus dem Inneren von Hohlkörpern, insbesondere von Thromben und Embolien aus Blutgefässen, auch Verschlussentfernung genannt, besteht darin, dass zumindest ein Führungsdraht in den Hohlkörper eingeführt wird und vor dem Einführen des Abbaukatheters ein Schleusenkatheter, der gemäss den vorgenannten Ausführungen ausgebildet ist, entlang des eingeführten Führungsdrahtes bis vor das zu entfernende Material eingeführt wird und der Querschnitt des Hohlkörpers mittels des Schleusenkatheters wenigstens teilweise verschlossen wird, wobei das zu entfernende Material von einem Abbaukatheter, der gemäss den vorgenannten Ausführungen ausgebildet ist, abgebaut, von diesem angesaugt und durch die flexible Hülle abgesaugt wird. Mit diesem Verfahren wird eine Entfernung der im Hohlkörper befindlichen Ablagerung ermöglicht, ohne dass sich Teile des abgebauten Materials durch den Blutfluss an weiter entfernten Stellen im Hohlkörper ablagern können, welche dort zu neuen Verengungen oder Verschlüssen des Hohlkörpers führen könnten.

Zweckmässigerweise wird dabei zuerst der Schleusenkatheter bis vor das zu entfernende Material in den Hohlkörper eingeführt und der Hohlkörper wird durch radiales Aufweiten des mit dem distalen Ende des Schleusenkatheters verbundenen Endstückes wenigstens teilweise abgeschlossen. Hierauf wird der Abbaukatheter von der dem Schleusenkatheter entgegengesetzten Seite her bis vor das zu entfernende Material in den Hohlkörper eingeführt und dann das zu entfernende Material unter Rotation der Förderschraube in die wenigstens eine Eintrittsöffnung des Stators angesaugt, fragmentiert und durch die flexible Hülle hinausbefördert.
Der Schleusenkatheter wird vorzugsweise entgegen der Blutflussrichtung in den Hohlkörper eingeführt. Beim Abbau des zu entfernenden Materials durch den Abbaukatheter gelöstes und vom Blutfluss transportiertes Material wird im Endstück des Schleusenkatheters aufgefangen und gesammelt. Nach dem Entfernen der Ablagerung im Hohlkörper kann mit dem Abbaukatheter das aufgeweitete Endstück des Schleusenkatheters ausgeräumt, d. h. vom sich im Endstück angesammelten Material der abgebauten Ablagerung befreit werden. Anschliessend wird das Endstück des Schleusenkatheters vorteilhaft zusammengefaltet und der Schleusenkatheter sowie der Abbaukatheter in jeweils entgegengesetzten Richtungen aus dem Hohlkörper herausgezogen.

In einem weiteren nicht beanspruchten Verfahren wird mit dem Abbaukatheter Material aus einem bereits im Hohlkörper temporär oder permanent angeordneten Filter ausgeräumt. Bei gewissen Anwendungen wird ein Filter im Hohlkörper positioniert, der den Hohlkörper flüssigkeitsdurchlässig, aber partikeldicht abschliesst. Dieser Filter kann temporär, z. B. vor oder während einem chirurgischen Eingriff, oder permanent, d. h. über einen längeren Zeitraum, im Hohlkörper angeordnet werden. Um den Blutdurchfluss durch den im Hohlkörper temporär oder permanent angeordneten Filter sicherzustellen, muss dieser entweder entfernt werden, wobei bei der Entfernung sich in diesem Filter angesammeltes Material im Hohlkörper und somit im Blutfluss verteilen kann, oder mittels einem Abbaukatheter ausgeräumt werden. In diesem Fall wird vorteilhaft die erfindungsgemässe Vorrichtung verwendet. Zuerst wird beispielsweise der Schleusenkatheter, vorteilhaft entgegen dem Blutfluss beziehungsweise in Gegenrichtung des Blutflusses, bis vor den im Hohlkörper temporär oder permanent angeordneten Filter herangeführt und das Endstück des Schleusenkatheters zum zumindest teilweisen Abschliessen des Hohlkörpers aufgeweitet. Anschliessend wird von der anderen Seite her der Abbaukatheter in den Hohlkörper eingeführt und der im Hohlkörper temporär oder permanent angeordnete Filter ausgeräumt. Allfällig dabei durch den Blutfluss mittransportiertes Material aus dem im Hohlkörper temporär oder permanent angeordneten Filter wird im aufgeweiteten Endstück des Schleusenkatheters aufgefangen und kann beim Entfernen des Schleusenkatheters aus dem Hohlkörper mit diesem ebenfalls aus dem Hohlkörper entfernt werden.

Ein weiteres alternatives nicht beanspruchtes Verfahren besteht darin, dass wieder zuerst der Schleusenkatheter bis vor das zu entfernende Material in den Hohlkörper eingeführt und der Hohlkörper durch radiales Aufweiten des Endstückes wenigstens teilweise abgeschlossen wird. Hierauf wird der Abbaukatheter koaxial durch einen zentralen Kanal durch den Schleusenkatheter hindurch bis vor das zu entfernende Material in den Hohlkörper eingeführt und dann das zu entfernende Material unter Rotation der Förderschraube in die wenigstens eine Eintrittsöffnung des Stators angesaugt, fragmentiert und durch die flexible Hülle hinausbefördert. Bei diesem Verfahren werden der Schleusenkatheter und der Abbaukatheter vorzugsweise an der gleichen Stelle in den Hohlkörper und somit in den Körper eingeführt, so dass nur eine Einführöffnung, z. B. im Körper des Patienten, erstellt werden muss.

Zweckmässigerweise wird zunächst ein gemeinsamer Führungsdraht in den Hohlkörper eingeführt und dann sowohl der Abbaukatheter als auch der Schleusenkatheter über den gemeinsamen Führungsdraht miteinander oder zeitlich versetzt von einer Seite her oder gegeneinander, d. h. von zwei Seiten her in den Hohlkörper eingeführt. Der Führungsdraht wird vorgängig z. B. mit Röntgenunterstützung in den Hohlkörper eingeführt und führt den Schleusenkatheter wie auch den Abbaukatheter beim Einführen in den Hohlkörper. Der gemeinsame Führungsdraht stellt sicher, dass der Abbaukatheter und der Schleusenkatheter aufeinander treffen. Der Abbaukatheter und der Schleusenkatheter können somit optimal miteinander zusammenwirken, wobei die Gefahr des Verlassens eines Blutgefässes verhindert ist.

In einem alternativen Verfahrensschritt werden ein erster Führungsdraht für den Schleusenkatheter und ein zweiter Führungsdraht für den Abbaukatheter entgegen der Einführrichtung des ersten Führungsdrahtes in den Hohlkörper eingeführt. Dann wird der Schleusenkatheter über den ersten Führungsdraht an der gewünschten Stelle im Hohlkörper positioniert. Anschliessend wird der erste Führungsdraht zumindest teilweise zurückgezogen und dann der zweite Führungsdraht zumindest bereichsweise in den Schleusenkatheter eingeschoben und nun der Abbaukatheter entlang des zweiten Führungsdrahtes sicher in Richtung des Schleusenkatheters im Hohlkörper eingeführt. Die in solchen Verfahren üblicherweise verwendeten Führungsdrähte weisen ein flexibles Ende auf, welches ein Finden des entsprechenden Hohlkörpers z. B. bei Abzweigungen erleichtern. Es ist daher teilweise schwierig den Schleusenkatheter und/oder den Abbaukatheter über dieses flexible Ende des Führungsdrahtes in den Hohlkörper einzuführen. Durch das zumindest bereichsweise Einschieben des zweiten Führungsdrahtes in den Schleusenkatheter wird in einer weiteren vorteilhaften Weise sichergestellt, dass der Abbaukatheter und der Schleusenkatheter aufeinander treffen und somit der Abbaukatheter und der Schleusenkatheter optimal miteinander zusammenwirken können.

Das radiale Aufweiten des mit dem proximalen Ende des Schleusenkatheters verbundenen Endstückes erfolgt beispielsweise mittels eines aufblasbaren Ballons, mittels eines selbstexpandierenden Mechanismus oder durch den Abbaukatheter, was einen wenigstens teilweisen Verschluss des Hohlkörpers durch das aufgeweitete Endstück gewährleistet.

Weitere Ausbildungen der Erfindung sind in den Figuren und in den abhängigen Patentansprüchen angegeben. Die Bezugszeichenliste ist Bestandteil der Offenbarung.

Anhand von Figuren wird die Erfindung symbolisch und beispielhaft näher erläutert.

Die Figuren werden zusammenhängend und übergreifend beschrieben. Gleiche Bezugszeichen bedeuten gleiche Bauteile.

Es zeigen dabei:
- Fig.1: Ein durch einen Thrombus verstopftes Blutgefäss, mit aus verschiedenen Richtungen in das Blutgefäss eingeführtem Abbaukatheter und Schleusenkatheter, zu Beginn der Behandlung,
- Fig. 2: das Blutgefäss gemäss Fig.1, nach dem Entfernen des Thrombus
- Fig. 3: eine Variante des in den Fig. 1 und 2 dargestellten Verfahrens,
- Fig. 4: den aus den Fig. 1 bis 3 ersichtlichen Abbaukatheter, in vergrösserter Darstellung, im Längsschnitt,
- Fig. 5: eine Variante des in der Fig. 4 dargestellten Abbaukatheters im Längsschnitt,
- Fig. 6 bis 10: unterschiedliche Ausführungsformen des am distalen Ende des Abbaukatheters angeordneten Stators und
- Fig. 11: eine weitere Variante des in den Fig. 1 und 2 dargestellten Verfahrens.

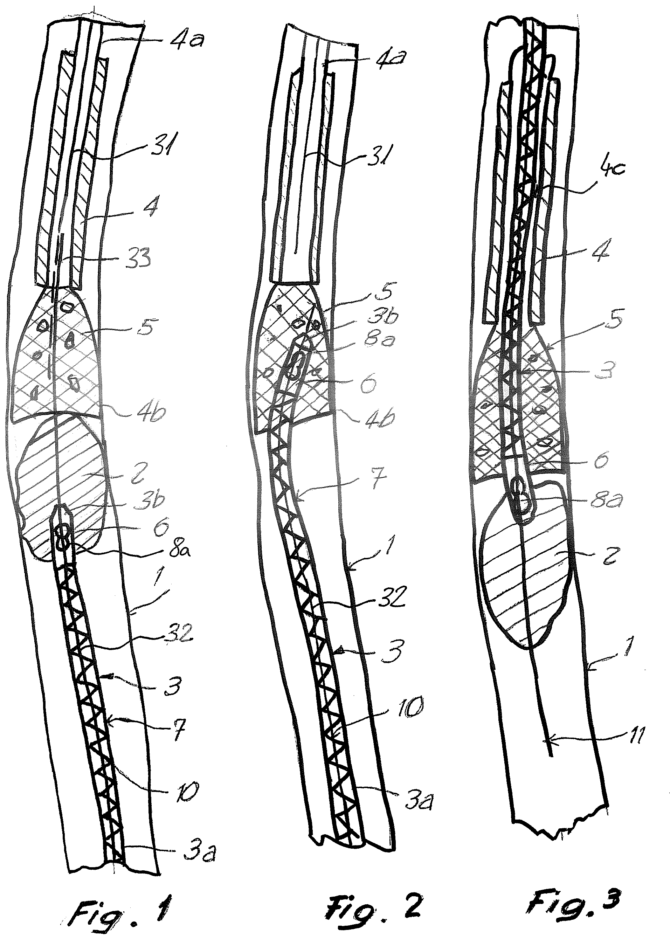

Die Figuren 1 bis 3 zeigen schematisch einen Hohlkörper 1, bspw. eine Vene oder Arterie im Längsschnitt. In dem Hohlkörper 1 befindet sich eine den Durchfluss des Blutes behindernde Ablagerung. Diese kann beispielsweise ein Thrombus 2 oder eine Embolie sein, deren Material entfernt werden muss, damit die Blutzirkulation wieder richtig funktioniert und die Körperorgane genügend mit den nötigen Stoffen versorgt werden.

In den Figuren 1 und 2 ist jeweils eine Vorrichtung zum Entfernen und Hinausfördern von einem Thrombus 2 aus dem Innern des Hohlkörpers 1 dargestellt. Die Vorrichtung umfasst einen in den Hohlkörper 1 einführbaren Abbaukatheter 3 und einen, ebenfalls in denselben Hohlkörper 1 einführbaren Schleusenkatheter 4. Der Schleusenkatheter 4 weist ein proximales Ende 4a und ein distales Ende 4b auf. Das distale Ende 4b des Schleusenkatheters 4 ist mit einem radial verformbaren, trichterförmigen Endstück 5 aus flüssigkeitsdurchlässigem Material verbunden.

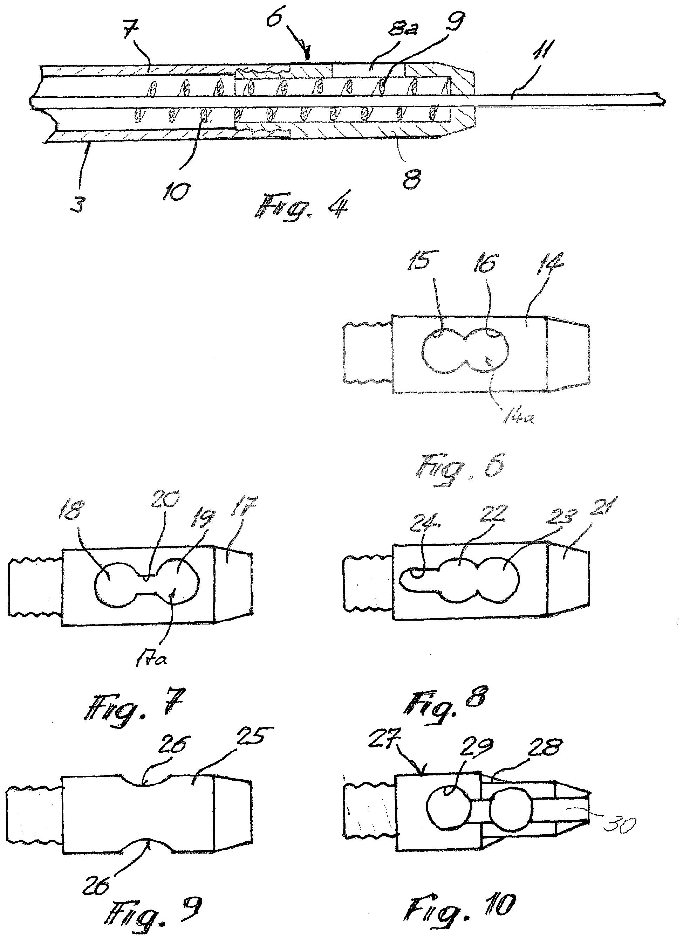

Der Abbaukatheter 3 weist ebenfalls ein proximales Ende 3a und ein distales Ende 3b auf. Am distalen Ende 3b befindet sich ein Arbeitskopf 6. Der Arbeitskopf 6 ist mit einer flexiblen, schlauchförmigen Hülle 7 verbunden. Der Arbeitskopf 6 des Abbaukatheters 3 weist zum Einführen in den Bereich des trichterförmigen Endstückes 5 des Schleusenkatheters 4 einen Aussendurchmesser auf, der kleiner als der Innendurchmesser des erweiterten Endstückes 5 ist. Wie nachfolgend noch im Zusammenhang mit den Figuren 4 bis 10 im Detail beschrieben wird, weist der Arbeitskopf 6 des Abbaukatheters 3 einen hülsenförmigen Stator 8 und einen zentral darin angeordneten, relativ zum Stator 8 in Drehung versetzbaren Rotor 9 auf, wobei der Stator 8 an seinem Umfang mit wenigstens einer seitlichen Eintrittsöffnung 8a versehen ist.

Bei dem in den Figuren 1 und 2 dargestellten Verfahren wird zunächst ein erster Führungsdraht 31 in den Hohlkörper 1 bis etwa zum Thrombus 2 eingeführt (siehe gestricheltes Ende des ersten Führungsdrahtes 31). Anschliessend wird der Schleusenkatheter 4 über den ersten Führungsdraht 31 von der gleichen Seite her ebenfalls in den Hohlkörper 1 eingeführt und bis unmittelbar vor den zu entfernenden Thrombus 2 vorgeschoben. Beim Einführen wird das Endstück 5 beispielsweise durch eine zerstörbare Hülle im Durchmesser komprimiert. Ist die gewünschte Position des Endstückes 5 des Schleusenkatheters 4 erreicht, so wird die Hülle entfernt und das Endstück 5 kann sich infolge der eigenen Elastizität schirmartig entfalten. Alternativ wird das Endstück 5 durch zusätzliche Hilfe von aussen, z. B. mittels eines aufblasbaren Ballons, mittels eines selbstexpandierenden Mechanismus oder durch den Abbaukatheter radial aufgeweitet. Dabei wird der Hohlkörper 1 durch das Endstück 5 des Schleusenkatheters 4 wenigstens teilweise abgeschlossen. Durch das flüssigkeitsdurchlässige Material des Endstückes 5 kann jedoch weiterhin das Blut durch den Hohlkörper 1 fliessen. Es werden nur die im Blutstrom enthaltenen festen Bestandteile bzw. Partikel von dem Endstück 5 des Schleusenkatheters 4 herausgefiltert.

Nachdem der Schleusenkatheter 4 korrekt und an der vorgesehenen Position festgelegt ist oder vor der Positionierung des Schleusenkatheters 4 wird von der dem Schleusenkatheter 4 gegenüberliegenden Seite des Thrombus 2 ein zweiter Führungsdraht 32 in den Hohlkörper 1 eingeführt und durch den Thrombus 2 hindurchgeführt. Der erste Führungsdraht 31 wird nun ein wenig zurückgezogen und dann der zweite Führungsdraht 32 weiter vorgeschoben, so dass dessen freies Ende 33 bereichsweise in den Schleusenkatheter 4 eindringt. Nun wird der Abbaukatheter 3 entlang des zweiten Führungsdrahtes 32 in den Hohlkörper 1 von der dem Schleusenkatheter 4 gegenüberliegenden Seite eingeschoben.

Wie insbesondere aus der Figur 4 ersichtlich, umfasst der Arbeitskopf 6 des Abbaukatheters 3 einen hülsenförmigen Stator 8 und eine koaxial darin gelagerte Förderschraube 10, die entweder einstückig oder gekoppelt mit einem ebenfalls als Förderschraube wirkenden Rotor 9 verbunden ist. Im vorliegenden Beispiel bildet der distale, im Stator 8 rotierende Endabschnitt der Förderschraube 10 den Rotor 9 aus. Der Stator 8 ist mit wenigstens einer seitlichen Eintrittsöffnung 8a versehen und weist an seinem distalen Ende einen sich zum freien Ende hin verjüngenden Abschnitt auf. Der Stator 8 ist an seinem proximalen Ende mit einer schlauchartigen, flexiblen Hülle 7 verbunden.

Wie in der Figur 1 gezeigt, wird durch den Arbeitskopf 6 des Abbaukatheters 3 nun der Thrombus 2 abgebaut. Teile des Thrombus 2 werden infolge eines, durch die Rotation der Förderschraube 10 erzeugten Unterdrucks durch die Eintrittsöffnung 8a am Stator 8 ins Innere des Arbeitskopfes 6 gezogen und dabei durch die Relativverdrehung zwischen Rotor 9 und Stator 8 an den Kanten der Eintrittsöffnung 8a abgeschert, dabei fragmentiert bzw. zerkleinert sowie über die Förderschraube 10 durch die flexible Hülle 7 abgeführt. Der Schleusenkatheter 4 federt während des Abbauvorgangs mit seinem Endstück 5 kontinuierlich auf, so dass der Hohlkörper 1 dabei sicher verschlossen ist.

Allfällige sich lösende und nicht in den Arbeitskopf 6 des Abbaukatheters 3 eingesaugte Bestandteile des Thrombus 2, welche der Blutstrom bzw. Blutfluss weiterbefördert, werden im aufgefederten trichterförmigen Endstück 5 des Schleusenkatheters 4 aufgefangen. Nachdem der ganze Thrombus 2 mittels des Abbaukatheters 3 entfernt ist, kann durch weiteres Vorschieben des Abbaukatheters 3 auch das trichterförmige Endstück 5 des Schleusenkatheters 4 ausgeräumt und von den sich darin verfangenen Partikeln befreit werden. Dies ist aus Figur 2 deutlich ersichtlich. Zum Ausräumen des Endstückes 5 des Schleusenkatheters 4 kann der zweite Führungsdraht 32 ein wenig zurückgezogen werden, so dass der Arbeitskopf 6 des Abbaukatheters 3 freier innerhalb des Endstückes 5 des Schleusenkatheters 4 bewegbar ist. Anschliessend werden der Abbaukatheter 3 und der zweite Führungsdraht 32 sowie der Schleusenkatheter 4 und der erste Führungsdraht 31 in jeweils entgegengesetzter Richtung aus dem Hohlkörper 1 herausgezogen und aus diesem entfernt.

Bei dem in der Figur 3 dargestellten Verfahren werden im Unterschied zu dem Verfahren gemäss den Darstellungen in den Figuren 1 und 2 der Abbaukatheter 3 sowohl als auch der Schleusenkatheter 4 von derselben Seite her in den Hohlkörper 1 eingeführt. Dabei wird zunächst der Schleusenkatheter 4 entlang eines vorgängig eingeführten Führungsdrahtes 11 bis vor den zu entfernenden Thrombus 2 in den Hohlkörper 1 eingeführt. Anschliessend wird das trichterförmige Endstück 5 des Schleusenkatheters 4 radial aufgeweitet, sodass der Hohlkörper 1 zumindest teilweise abgeschlossen ist. Nachdem das Endstück 5 festgelegt ist, wird der Abbaukatheter 3 durch einen zentralen Kanal 4c im Schleusenkatheter 4 eingeführt und der Abbau des Thrombus 2 von dieser Seite her begonnen. Allfällige sich lösende und durch den Blutfluss abgeschwemmte Partikel werden, analog dem im Zusammenhang mit den Figuren 1 und 2 beschriebenen Verfahren, durch das trichterförmige Endstück 5 des Schleusenkatheters 4 aufgefangen. Beim anschliessenden Zurückziehen des Abbaukatheters 3 durch den Schleusenkatheter 4 kann das trichterförmige Endstück 5 des Schleusenkatheters 4 ausgeräumt und sich darin befindliche Partikel entfernt werden. Bei diesem Verfahren sind der Abbaukatheter 3 und der Schleusenkatheter 4 auf einem gemeinsamen Führungsdraht 11 geführt.

Bei einer Variante, bei der der Abbaukatheter gleichzeitig auch zur Aufweitung des Endstückes des Schleusenkatheters benutzt wird, ist dieses im radial innen liegenden Bereich so geformt, dass es dort schmäler als der Aussendurchmesser des Abbaukatheters ist. Wird der Abbaukatheter nun durch sanften Druck axial nach vorne geschoben, dehnt er dabei das Endstück des Schleusenkatheters auf.

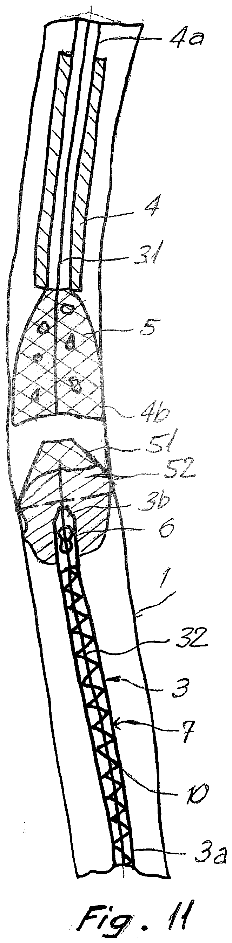

Bei dem in der Figur 11 dargestellten Verfahren wird mit der einen Schleusenkatheter 4 und einen Abbaukatheter 3 umfassenden Vorrichtung Material 52 aus einem bereits im Hohlkörper 1 temporär oder permanent angeordneten Filter 51 ausgeräumt. Das Verfahren zum Positionieren des Schleusenkatheters 4 und Anordnen des Abbaukatheters 3 entspricht im Wesentlichen dem im Zusammenhang mit den Figuren 1 und 2 beschriebenen Verfahren.

Bei der Entfernung des im Filter 51 befindlichen Materials werden allfällig vom Blutstrom transportierte Partikel im aufgeweiteten Endstück 5 des Schleusenkatheters 4 aufgefangen.

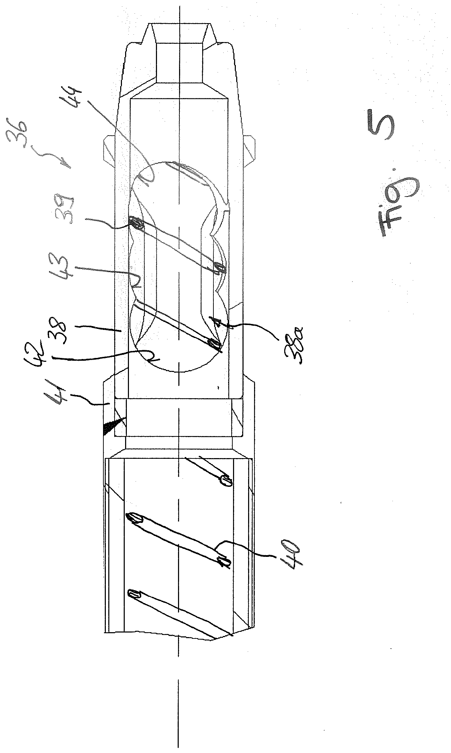

In der Figur 5 ist eine Variante für einen Arbeitskopf 36 eines Abbaukatheters 3 dargestellt. Der Arbeitskopf 36 weist neben dem Stator 38 ein zusätzliches, hier aussenliegendes Rotorelement 41 auf. Der Stator 38 weist zudem mehrere seitliche Eintrittsöffnungen 38a auf, die jeweils von drei, in Bezug auf die Längsachse des Abbaukatheters 3 axial hintereinander angeordnete kreisrunde Löcher 42, 43 und 44 gebildet sind. Die Kreisumfänge der Löcher 42, 43 und 44 überschneiden sich. Durch die Ausgestaltung der Löcher 42, 43 und 44 weist jede Eintrittsöffnung 38a jeweils sechs Scherkanten auf, die vorteilhaft als Schneidkanten ausgebildet sind.

In einer hier nicht dargestellten Variante ist das zusätzliche Rotorelement 41 innenseitig des Stators 38 vorgesehen.

Die Figuren 6 bis 10 zeigen unterschiedliche Ausführungsformen des Stators.

Beim Stator 14 gemäss Figur 6 sind zwei kreisrunde Löcher 15, 16 axial so hintereinander angeordnet, dass sich deren Kreisumfänge teilweise überschneiden. Diese seitliche Eintrittsöffnung 14a hat somit die Form einer Acht und weist vier Scherkanten auf.

Bei dem aus Figur 7 ersichtlichen Stator 17 sind ebenfalls zwei Löcher 18, 19 axial hintereinander angeordnet, welche die seitliche Eintrittsöffnung 17a ausbilden. In diesem Fall überschneiden sich deren Kreisumfänge jedoch nicht. Die beiden Löcher 18, 19 sind durch einen in axialer Richtung verlaufenden Schlitz 20 miteinander verbunden.

Der aus Figur 8 ersichtliche Stator 21 unterscheidet sich von der Ausführung gemäss der Figur 7 dadurch, dass ein Schlitz 24 die beiden Löcher 22, 23 zum proximalen Ende hin axial überragt. Der Schlitz 24 und die beiden Löcher 22, 23 bilden die seitliche Eintrittsöffnung 21 a. Dieser Schlitz 24 ist besonders günstig bei sehr zähem und faserigem abzutragendem Material, da er durch die zusätzlich zur Verfügung stehenden Scherkanten ein gutes Abscheren und somit Fragmentierten des angesaugten Materials bewirkt.

Wie der aus Figur 9 ersichtliche Stator 25 zeigt, können auch zwei einander etwa diametral gegenüberliegende Eintrittsöffnungen 26 vorgesehen werden, welche vorteilhaft spiegelbildlich zueinander angeordnet sind.

Der aus Figur 10 ersichtliche Stator 27 unterscheidet sich von den in den Figuren 5 bis 9 dargestellten Ausführungen dadurch, dass er gegen sein distales Ende im Durchmesser abgestuft ist. Der an seinem distalen Ende verringerte Durchmesser des Stators 27 ermöglicht ein leichteres Eindringen des Abbaukatheters 3 in das zu entfernende Material.

Die aus den Figuren 5 bis 10 ersichtlichen Ausbildungen der seitlichen Eintrittsöffnungen am Stator sind besonders zweckmässig im Zusammenwirken mit dem trichterförmigen Endstück 5 des Schleusenkatheters 4. So kann ein Verhaken oder Verklemmen des Stators im Material des Endstückes 5 weitgehend verhindert werden.

**Bezugszeichenliste**

| | | | |
|---|---|---|---|
| 1 | Hohlkörper | 21 | Stator |
| 2 | Thrombus | 21a | Eintrittsöffnung |
| 3 | Abbau katheter | 22 | Loch |
| 3a | proximales Ende v. 3 | 23 | Loch |
| 3b | distales Ende v. 3 | 24 | Schlitz |
| 4 | Schleusenkatheter | 25 | Stator |
| 4a | proximales Ende v. 4 | 26 | Eintrittsöffnung |
| 4b | distales Ende v. 4 | 27 | Stator |
| 4c | zentraler Kanal v. 4 | 28 | Absatz |
| 5 | Endstück | 29 | Loch |
| 6 | Arbeitskopf | 30 | Schlitz |
| 7 | flexible Hülle | 31 | 1. Führungsdraht |
| 8 | Stator | 32 | 2. Führungsdraht |
| 8a | Eintrittsöffnung | 33 | freies Ende v. 32 |
| 9 | Rotor | | |
| 10 | Förderschraube | 38 | Stator |
| 11 | Führungsdraht | 38a | Eintrittsöffnung |
| | | 39 | Rotor |
| | | 40 | Förderschraube |
| 14 | Stator | 41 | Rotorelement |
| 14a | Eintrittsöffnung | 42 | Loch |
| 15 | Loch | 43 | Loch |
| 16 | Loch | 44 | Loch |
| 17 | Stator | | |
| 17a | Eintrittsöffnung | 51 | Filter |
| 18 | Loch | 52 | Material |
| 19 | Loch | | |
| 20 | Schlitz | | |

## Patentansprüche

1. Vorrichtung zum Entfernen und Hinausfördern von entfernbarem Material, wie Ablagerungen aus dem Innern von Hohlkörpern (1), insbesondere von Thromben (2) und Embolien aus Blutgefässen oder dergleichen, mit einem in den Hohlkörper (1) einführbaren Abbaukatheter (3) und einem ebenfalls in denselben Hohlkörper (1) einführbaren Schleusenkatheter (4), wobei der Abbaukatheter (3) und der Schleusenkatheter (4) je ein proximales Ende (3a, 4a) und ein distales Ende (3b, 4b) aufweisen, der Schleusenkatheter (4) an seinem distalen Ende (4b) mit einem radial verformbaren, trichterförmigen, sich gegen sein distales Ende im Querschnitt erweiternden Endstück (5) aus flüssigkeitsdurchlässigem Material verbunden ist und der Abbaukatheter (3) einen Arbeitskopf (6) und eine damit verbundene flexible Hülle (7) aufweist, wobei der Arbeitskopf (6) des Abbaukatheters (3) zum Einführen in den Bereich des trichterförmigen Endstückes (5) des Schleusenkatheters (4) einen Aussendurchmesser aufweist, der kleiner als der Innendurchmesser des erweiterten Endstückes ist, **dadurch gekennzeichnet, dass** der Arbeitskopf (6) des Abbaukatheters (3) einen hülsenförmigen Stator (8, 14, 17, 21, 25, 27, 38) und einen zentral darin angeordneten, relativ zum Stator (8, 14, 17, 21, 25, 27, 38) in Drehung versetzbaren Rotor (9, 39) aufweist, der Stator (8, 14, 17, 21, 25, 27, 38) an seinem Umfang mit wenigstens einer Eintrittsöffnung (8a, 14a, 17a, 21a, 38a) versehen ist und der Rotor (9, 39) mit einer Förderschraube (10, 40) verbunden oder einstückig ausgebildet ist, wobei die wenigstens eine seitliche Eintrittsöffnung (8a, 14a, 17a, 21a, 38a) des Stators (8, 14, 17, 21, 25, 27, 38) als wenigstens zwei, in Bezug auf die Längsachse des Abbaukatheters (3) axial hintereinander angeordnete kreisrunde Löcher (15, 16; 18, 19; 22, 23, 42, 43, 44) ausgebildet ist.

2. Vorrichtung nach Anspruch 1, **dadurch gekennzeichnet, dass** der Schleusenkatheter (4) einen zentralen Kanal (4c) aufweist, durch den der Abbaukatheter (3) so weit einführbar ist, dass der Arbeitskopf (6) des Abbaukatheters (3) im Abbauzustand bis in den Bereich des trichterförmigen Endstückes (5) ragt.

3. Vorrichtung nach Anspruch 1, **dadurch gekennzeichnet, dass** sich die Kreisumfänge der Löcher (15, 16, 18, 19, 22, 23, 42, 43, 44) überschneiden, **oder dass** die Löcher (18, 19, 22, 23) durch einen in axialer Richtung verlaufenden, im Wesentlichen zentrisch zu den Löchern (18, 19) angeordneten Schlitz (20, 24) miteinander verbunden sind, wobei vorzugsweise sich der Schlitz (24) in Richtung zum proximalen Ende des Abbaukatheters (3) axial über die Löcher (22, 23) hinaus erstreckt.

4. Vorrichtung nach einem der vorhergehenden Ansprüche, **dadurch gekennzeichnet, dass** am Stator (8, 25) des Abbaukatheters (3) jeweils zwei einander etwa diametral gegenüberliegend angeordnete, seitliche Eintrittsöffnungen (26) vorgesehen sind.

5. Vorrichtung nach einem der vorhergehenden Ansprüche, **dadurch gekennzeichnet, dass** der Stator (27) des Arbeitskopfes (6) im Aussendurchmesser so abgestuft ist, dass er sich zu seinem freien distalen Ende hin verjüngt.

6. Vorrichtung nach einem der vorhergehenden Ansprüche, **dadurch gekennzeichnet, dass** das Endstück (5) des Schleusenkatheters (4) im aufgeweiteten Zustand des Endstückes (5) eine Sieb- oder Gitterstruktur aufweist, und vorzugsweise schirmartig aufweitbar ist.

7. Vorrichtung nach Anspruch 6, **dadurch gekennzeichnet, dass** das Endstück (5) des Schleusenkatheters (4) mittels eines aufblasbaren Ballons, mittels eines selbstexpandierenden Mechanismus oder durch den Abbaukatheter (3) radial aufweitbar ist.

8. Vorrichtung nach einem der vorhergehenden Ansprüche, **dadurch gekennzeichnet, dass** der Arbeitskopf (6) des Abbaukatheters (3) ein zusätzliches, relativ zum Stator (38) rotierendes Rotorelement (41) aufweist, das vorzugsweise innenseitig des Stators (38) angeordnet ist.

9. Vorrichtung nach einem der vorhergehenden Ansprüche, **dadurch gekennzeichnet, dass** die Kanten der Löcher (15, 16; 18, 19; 22, 23, 42, 43, 44) als Schneidkanten ausgebildet sind.

10. Abbaukatheter für eine Vorrichtung nach einem der vorhergehenden Ansprüche zum Entfernen und Hinausfördern von entfernbarem Material, wie Ablagerungen aus dem Innern von Hohlkörpern (1), insbesondere von Thromben (2) und Embolien aus Blutgefässen oder dgl., mit einem Arbeitskopf (6) und einer damit verbundenen flexiblen Hülle (7), in welcher eine Förderschraube (10, 40) vorgesehen ist, wobei der Arbeitskopf (6) des Abbaukatheters (3) einen hülsenförmigen Stator (8, 14, 17, 21, 25, 27, 38) aufweist, der an seinem Umfang mit wenigstens einer seitlichen Eintrittsöffnung (8a, 14a, 17a, 21a, 38a) versehen ist, **dadurch gekennzeichnet, dass** der hülsenförmige Stator (8, 14, 17, 21, 25, 27, 38) einen zentral darin angeordneten, relativ zum Stator (8, 14, 17, 21, 25, 27, 38) in Drehung versetzbaren Rotor (9, 39) aufweist, der mit der Förderschraube (10, 40) verbunden oder einstückig ausgebildet ist, wobei die wenigstens eine seitliche Eintrittsöffnung (8a, 14a, 17a, 21a, 38a) des Stators (8, 14, 17, 21, 25, 27, 38) als jeweils wenigstens zwei, in Bezug auf die Längsachse des Abbaukatheters axial hintereinander angeordnete kreisrunde Löcher (15, 16; 18, 19; 22, 23, 42, 43, 44) ausgebildet ist.

11. Abbaukatheter nach Anspruch 10, **dadurch gekennzeichnet, dass** ein in axialer Richtung verlaufender, im Wesentlichen zentrisch zu den Löchern (18, 19) angeordneter Schlitz (20, 24) vorgesehen ist, der mit zumindest einem der Löchern (18, 19) verbunden ist.

12. Abbaukatheter nach Anspruch 11, **dadurch gekennzeichnet, dass** die Löcher (18, 19) durch den Schlitz (20) miteinander verbunden sind.

13. Abbaukatheter nach einem der Ansprüche 11 oder 12, **dadurch gekennzeichnet, dass** sich der Schlitz (24) in Richtung zum proximalen Ende des Abbaukatheters (3) axial über die Löcher (22, 23) hinaus erstreckt.

14. Abbaukatheter nach einem der Ansprüche 10 bis 13, **dadurch gekennzeichnet, dass** sich die Kreisumfänge der Löcher (15, 16, 18, 19, 22, 23, 42, 43, 44) überschneiden.

15. Abbaukatheter nach einem der Ansprüche 10 bis 14, **dadurch gekennzeichnet, dass** der Stator (27) des Arbeitskopfes (6) im Aussendurchmesser so abgestuft ist, dass er sich zu seinem freien distalen Ende hin verjüngt.

## Claims

1. A device for removing and extracting removable material such as deposits from inside hollow bodies (1), in particular for removing and extracting thrombi (2) and emboli from blood vessels or the like, having a reducing catheter (3) which can be inserted into the hollow body, and a sheathing catheter (4) which can likewise be inserted into the same hollow body (1), wherein the reducing catheter (3) and the sheathing catheter (4) each have a proximal end (3a, 4a) and a distal end (3b, 4b), the distal end (4b) of the sheathing catheter (4) being connected to a radially deformable, funnel-shaped end piece (5) which is produced from liquid-permeable material and which widens in cross-section towards its distal end, and the reducing catheter (4) has a working head (6) and a flexible sleeve (7) connected thereto, wherein the working head (6) of the reducing catheter (3) has an external diameter which is smaller than the internal diameter of the widened end piece for insertion into the region of the funnel-shaped end piece (5) of the sheathing catheter (4), **characterized in that** the working head (6) of the reducing catheter (3) has a sleeve-shaped stator (8, 14, 17, 21, 25, 27, 38) and a rotor (9, 39) which is centrally disposed therein and which is displaceable in rotation relative to the stator (8, 14, 17, 21, 25, 27, 38), the stator (8, 14, 17, 21, 25, 27, 38) is provided at its circumference with at least one inlet opening (8a, 14a, 17a, 21a, 38a) and the rotor (9, 39) is connected to or configured as one piece with a feed screw (10, 40), wherein the at least one lateral inlet opening (8a, 14a, 17a, 21a, 38a) of the stator (8, 14, 17, 21, 25, 27, 38) is configured as at least two circular holes (15, 16; 18, 19; 22, 23, 42, 43, 44) which are disposed axially in succession with respect to the longitudinal axis of the reducing catheter (3).

2. The device as claimed in claim 1, **characterized in that** the sheathing catheter (4) has a central duct (4c) through which the reducing catheter (3) can be inserted until, in the reducing state, the working head (6) of the reducing catheter (3) projects as far as the region of the funnel-shaped end piece (5).

3. The device as claimed in claim 1, **characterized in that** the circular circumferences of the holes (15, 16, 18, 19, 22, 23, 42, 43, 44) intersect, or **in that** the holes (18, 19, 22, 23) are connected together by a slot (20, 24) extending in the axial direction essentially centrally to the holes (18, 19), wherein preferably, the slot (24) extends axially in the direction of the proximal end of the reducing catheter (3) beyond the holes (22, 23).

4. The device as claimed in one of the preceding claims, **characterized in that** two respective approximately diametrically opposed lateral inlet openings (26) are provided on the stator (8, 25) of the reducing catheter (3).

5. The device as claimed in one of the preceding claims, **characterized in that** the external diameter of the stator (27) of the working head (6) is graduated in a manner such that it tapers towards its free distal end.

6. The device as claimed in one of the preceding claims, **characterized in that** when the end piece (5) of the sheathing catheter (4) is in the deployed state, the end piece (5) has a screen or lattice structure and preferably can be deployed in the manner of an umbrella.

7. The device as claimed in claim 6, **characterized in that** the end piece (5) of the sheathing catheter (4) can be radially deployed by means of an inflatable balloon, by means of a self-expanding mechanism or by means of the reducing catheter (3).

8. The device as claimed in one of the preceding claims, **characterized in that** the working head (6) of the reducing catheter (3) has an additional rotor element (41) which rotates relative to the stator (38) and which is preferably disposed inside the stator (38).

9. The device as claimed in one of the preceding claims, **characterized in that** the edges of the holes (15, 16; 18, 19; 22, 23, 42, 43, 44) are configured as cutting edges.

10. A reducing catheter for a device as claimed in one of the preceding claims, for removing and extracting removable material such as deposits from inside hollow bodies (1), in particular thrombi (2) and emboli from blood vessels or the like, having a working head (6) and a flexible sleeve (7) connected therewith, in which a feed screw (10, 40) is provided, wherein the working head (6) of the reducing catheter (3) has a sleeve-shaped stator (8, 14, 17, 21, 25, 27, 38) the circumference of which is provided with at least one lateral inlet opening (8a, 14a, 17a, 21a, 38a), **characterized in that** the sleeve-shaped stator (8, 14, 17, 21, 25, 27, 38) has a rotor (9, 39) which is centrally disposed therein and which is displaceable in rotation relative to the stator (8, 14, 17, 21, 25, 27, 38), which is connected to or configured as one piece with the feed screw (10, 40), wherein the at least one lateral inlet opening (8a, 14a, 17a, 21a, 38a) of the stator (8, 14, 17, 21, 25, 27, 38) is configured as at least two circular holes (15, 16; 18, 19; 22, 23, 42, 43, 44) which are disposed axially in succession with respect to the longitudinal axis of the reducing catheter.

11. The reducing catheter as claimed in claim 10, **characterized in that** a slot (20, 24) is provided which extends in the axial direction essentially centrally to the holes (18, 19) and which is connected to at least one of the holes (18, 19).

12. The reducing catheter as claimed in claim 11, **characterized in that** the holes (18, 19) are connected together via the slot (20).

13. The reducing catheter as claimed in claim 11 or claim 12, **characterized in that** the slot (24) extends axially through the holes (22, 23) in the direction towards the proximal end of the reducing catheter (3).

14. The reducing catheter as claimed in one of claims 10 to 13, **characterized in that** the circular circumferences of the holes (15, 16, 18, 19, 22, 23, 42, 43, 44) intersect.

15. The reducing catheter as claimed in one of claims 10 to 14, **characterized in that** the external diameter of the stator (27) of the working head (6) is graduated in a manner such that it tapers towards its free distal end.

## Revendications

1. Procédé d'élimination et d'évacuation de matière éliminable, comme des dépôts, depuis l'intérieur de corps creux (1), en particulier de thrombus et d'embolies de vaisseaux sanguins ou similaires, comportant un cathéter d'élimination (3) pouvant être introduit dans le corps creux et un cathéter sas (4) pouvant également être introduit dans le même corps creux (1), le cathéter d'élimination (3 et le cathéters sas (4) présentant chacun une extrémité proximale (3a, 4a) et une extrémité distale (3b, 4b) , le cathéter sas (4) étant raccordé par son extrémité distale (4b) à un embout (5) en forme d'entonnoir déformable radialement et dont la section transversale s'élargit vers son extrémité distale en matériau perméable aux liquides et le cathéter d'élimination (3) présentant une tête de travail (6) et une enveloppe souple (7) qui lui est raccordée, la tête de travail (6) du cathéter d'élimination (3) destinée à être introduite dans la zone de l'embout en forme d'entonnoir (5) du cathéter sas (4) présentant un diamètre extérieur qui est inférieur au diamètre intérieur de l'embout élargi, **caractérisé en ce que** la tête de travail (6) du cathéter d'élimination (3) présente un stator de forme tubulaire (8, 14, 17, 21, 25, 27, 38) et un rotor (9, 39) centré dedans et pouvant être mis en rotation par rapport au stator (8, 14, 17, 21, 25, 27, 38), le stator (8, 14, 17, 21, 25, 27, 38) étant pourvu sur sa circonférence d'au moins une ouverture d'entrée (8a, 14a, 17a, 21a, 38a) et le rotor (9, 39) étant raccordé à une vis transporteuse (10, 40) ou réalisé en une seule pièce, l'au moins une ouverture d'entrée latérale (8a, 14a, 17a, 21a, 38a) du stator (8, 14, 17, 21, 25, 27, 38) se présentant sous la forme d'au moins deux trous circulaires (15, 16; 18, 19; 22, 23, 42, 43, 44) disposés axialement les uns derrière les autres par rapport à l'axe longitudinal du cathéter d'élimination (3).

2. Dispositif selon la revendication 1, **caractérisé en ce que** le cathéter sas (4) présente un canal central (4c) dans lequel le cathéter d'élimination (3) peut être introduit jusqu'à ce que la tête de travail (6) du cathéter d'élimination (3) en état monté dépasse jusqu'au niveau de l'embout en forme d'entonnoir (5).

3. Dispositif selon la revendication 1, **caractérisé en ce que** les circonférences circulaires des trous (15, 16, 18, 19, 22, 23, 42, 43, 44) se coupent ou que les trous (18, 19, 22, 23) sont raccordés entre eux par une fente (20, 24) s'étendant dans le sens axial et pratiquée sensiblement de manière centrée par rapport aux trous (18, 19), la fente (4) s'étendant de préférence axialement en direction de l'extrémité proximale du cathéter d'élimination (3) au-delà des trous (22, 23).

4. Dispositif selon une des revendications précédentes, **caractérisé en ce qu'**il est prévu, au niveau du stator (8, 25) du cathéter d'élimination (3), respectivement deux ouvertures d'entrée latérales disposée de manière à être approximativement diamétralement opposées (26).

5. Dispositif selon une des revendications précédentes, **caractérisé en ce que** le stator (27) de la tête de travail (6) a un diamètre extérieur échelonné de manière à se rétrécir en direction de son extrémité distale libre

6. Dispositif selon une des revendications précédentes, **caractérisé en ce que** l'embout (5) du cathéter sas, dans l'état élargi de l'embout (5), présente une structure de tamis ou de grille et peut de préférence être élargi en forme de parapluie.

7. Dispositif selon la revendication 6, **caractérisé en ce que** l'embout (5) du cathéter sas (4) peut être élargi radialement au moyen d'un ballon gonflable, au moyen d'un mécanisme d'auto-expansion ou par le cathéter d'élimination (3).

8. Dispositif selon une des revendications précédentes, **caractérisé en ce que** la tête de travail (6) du cathéter d'élimination (3) présente un élément rotor (41) supplémentaire tournant par rapport au stator (38) et qui est disposé de préférence à l'intérieur du stator (38).

9. Dispositif selon une des revendications précédentes, **caractérisé en ce que** les bords des trous (15, 16; 18, 19; 22, 23, 42, 43, 44) se présentent sous forme d'arêtes de coupe.

10. Cathéter d'élimination pour un dispositif selon une des revendications précédentes d'élimination et d'évacuation de matière éliminable, comme des dépôts, depuis l'intérieur de corps creux (1), en particulier de thrombus et d'embolies de vaisseaux sanguins ou similaires, comportant une tête de travail (6) et une enveloppe flexible (7) qui lui est raccordée et dans laquelle est prévue une vis transporteuse (10, 40), la tête de travail (6) du cathéter d'élimination (3) présentant un stator de forme tubulaire (8, 14, 17, 21, 25, 27, 38) qui est pourvu sur sa circonférence d'au moins une ouverture d'entrée latérale (8a, 14a, 17a, 21a, 38a), **caractérisé en ce que** le stator de forme tubulaire (8, 14, 17, 21, 25, 27, 38) présente un rotor (9, 39) disposé dedans et pouvant être mis en rotation par rapport au stator (8, 14, 17, 21, 25, 27, 38) et qui est raccordé à la vis transporteuse (10, 40) ou réalisé en une seule pièce, l'au moins une ouverture d'entrée latérale (8a, 14a, 17a, 21a, 38a) du stator (8, 14, 17, 21, 25, 27, 38) se présentant sous la forme d'au moins deux trous circulaires (15, 16; 18, 19; 22, 23, 42, 43, 44) disposés axialement les uns derrière les autres par rapport à l'axe longitudinal du cathéter d'élimination (3).

11. Cathéter d'élimination selon la revendication 10, **caractérisé en ce qu'**une fente (20, 24) s'étendant dans le sens axial et pratiquée de manière à être sensiblement centrée par rapport aux trous (18, 19) est raccordée à au moins un des trous (18, 19).

12. Cathéter d'élimination selon la 11, **caractérisé en ce que** les trous (18, 19) sont raccordés entre eux par la fente (20).

13. Cathéter d'élimination selon une des revendications 11 ou 12, **caractérisé en ce que** la fente (24) s'étend axialement en direction de l'extrémité proximale du cathéter d'élimination (3) au-delà des trous (22, 23).

14. Cathéter d'élimination selon une des revendications 10 à 13, **caractérisé en ce que** les circonférences circulaires des trous (15, 16, 18, 19, 22, 23, 42, 43, 44) se coupent.

15. Cathéter d'élimination selon une des revendications 10 bis 14, **caractérisé en ce que** le stator (27) de la tête de travail (6) a un diamètre extérieur échelonné de manière à se rétrécir en direction de son extrémité distale libre.
